# EUROPEAN PATENT SPECIFICATION

(11) **EP 4 199 901 B1**
(45) Date of publication and mention of the grant of the patent: **04.11.2026**
(21) Application number: 21769282.1
(22) Date of filing: 19.08.2021
(51) Int. Cl.: A61K 9/00

(54) **EFFERVESCENT FORMULATION CONTAINING APOAEQUORIN**
BRAUSEFORMULIERUNG MIT APOAEQUORIN
FORMULATION EFFERVESCENTE CONTENANT DE L'APOAEQUORINE

(30) Priority: 20.08.2020 US 202063068084 P
(43) Date of publication of application: 28.06.2023
(73) Proprietor: Quincy Bioscience, LLC, Middleton, WI 53562 (US)
(72) Inventor: UNDERWOOD, Mark, Y., Verona, WI 53593-8655 (US)
(74) Representative: J A Kemp LLP
(86) International application number: PCT/US2021/046614
(87) International publication number: WO 2022/040380

(56) References cited:
- WO-A1-2009/114597
- WO-A1-2011/152875
- WO-A1-2018/058050
- US-A1- 2005 019 427

## Description

### CROSS-REFERENCE TO RELATED APPLICATION

This application claims the benefit of United States provisional patent application serial number 63/068,084 filed August 20, 2020.

### FIELD OF THE INVENTION

This invention relates generally to compositions useful for the maintenance of calcium homeostasis. In particular, this invention is directed to apoaequorin-containing effervescent compositions useful in preventing and/or alleviating diseases or symptoms associated with calcium imbalance.

### BACKGROUND OF THE INVENTION

Calcium is the fifth most abundant element in the human body and occurs mainly in the bone. More than 99% of the calcium in the body is stored in the skeleton, which constantly exchanges its supply with the remaining 1% dissolved in body fluids and soft tissue, such as the blood. The control of this exchange is largely dictated by the endocrine system which senses the concentration of ionized calcium in the plasma and directs calcium exchange to maintain this critical balance. Only a small fraction of the 1% of calcium in interstitial fluids and soft tissues is ionized and soluble. The remaining calcium in fluids and tissues is bound to proteins, particularly calcium-binding proteins (CaBPs). CaBPs are known to function in the maintenance of calcium homeostasis.

As the body requires specific concentrations of calcium ions to carry out requisite physiological processes, the maintenance of calcium homeostasis is of critical importance for bodily health. Proper ionic calcium concentrations in plasma and body fluids are understood by the medical community to be critical in bodily functions, including, but not limited to, neuronal excitability, muscle contraction, membrane permeability, cell division, hormone secretion and bone mineralization. A disruption in calcium homeostasis, i.e., a calcium imbalance, is associated with many diseases, syndromes and conditions, including, but not limited to, cancer, heart disease and neurodegenerative disease.

In the past, calcium channel antagonists, which block the flow of calcium between cell interiors and interstitial fluid, have been widely-prescribed as pharmaceutical agents useful in the prevention of calcium-related disorders including hypertension, angina, asthma, migraines and neural deterioration. For example, nimidopine has been found to improve clinical symptomatology and cognitive functions in dementia by alleviating a calcium imbalance which causes neural deterioration. However, many of these calcium channel antagonists have unwanted side effects including, but not limited to, malaise, fluid retention, heartburn, erratic heart rate, dizziness, upset stomach and, in rare cases, fainting, fever and excessive bleeding. The published international patent WO-2009114597 discloses a composition for treating a symptom or disorder associated with calcium imbalance, comprising: (a) an effective amount of apoaequorin; and (b) an acceptable solid carrier, such as a tablet.

Despite these advances, there is still a need for new and alternative compositions which alleviate or prevent calcium imbalance. In particular, pharmaceutical or nutraceutical compositions which have reduced side effects as compared to prior agents are desired and, if discovered, would meet a long felt need in the medical and nutritional health communities.

Further, those pharmaceutical or nutraceutical compositions must be administered to humans of all ages and health levels, which can often be a challenge. Particularly, patients are often reluctant to swallow pills, tablets, capsules, or other solid dosage medicament formulations, especially when the act of swallowing is problematic for that individual. For example, global hystericus and choking due to pharyngeal and esophageal motility problems, renders it painful to swallow and often results in aversion to swallowing the formulation. In addition, patients with pharyngitis and/or a markedly swollen or an otherwise severely irritated pharynx, such as due to a bacterial infection, often makes it difficult and/or impossible for the patient to swallow a solid medicament formulation. Patients may also be reluctant to ingest a medicament formulation due to its size, shape, and taste, psychological aversion to the act of ingestion, and/or personal choice not to swallow the formulation. Young patients are particularly problematic in this regard. However, patients under a medication regimen and/or in need of the therapeutic active ingredient in the formulation must self-administer, or be administered, the dose. Thus, there is an ongoing need to provide a better method of administering a therapeutically effective dose of a composition including formulations to treat calcium imbalance.

### SUMMARY OF THE INVENTION

The present invention provides various advantages over prior compositions and methods in that it provides for the general improvement of a subject's mental and physical health. The scope of the invention is defined by the claims.

A supplement in form of an effervescent tablet for treating a symptom or disorder associated with calcium imbalance comprising an effective amount of apoaequorin and an effervescent couple, the couple comprising an acidic component and an alkaline component; wherein the effervescent component comprises about 5% to about 85% of the total weight of the effervescent tablet.

A therapeutic effervescent composition for treating a symptom or disorder associated with calcium imbalance comprising: an effective amount of apoaequorin; an effective amount of vitamin D; at least one stimulant; and an effervescent system including at least one acid and at least one alkali compound; wherein the effervescent component comprises about 5% to about 85% of the total weight of the composition.

In a first aspect, the present invention is directed to supplements in the form of effervescent tablets for treating a symptom or disorder associated with calcium imbalance. Such methods of treatment include administering an effervescent tablet containing an effective amount of apoaequorin and an effervescent couple, where the couple includes an acidic component and an alkaline component. The acidic component may be citric acid, tartaric acid, malic acid, fumaric acid, adipic acid, succinic acids, or their salts, while the alkaline component may be sodium carbonate, sodium bicarbonate, sodium sesquicarbonate, potassium carbonate, potassium bicarbonate, potassium sesquicarbonate, magnesium carbonate, sodium glycine carbonate, L-lysine carbonate, arginine carbonate, amorphous calcium carbonate, ammonium carbonate, or ammonium bicarbonate.

In one embodiment, the supplement may further include at least one stimulant, which may be caffeine, yerba maté, ephedrine, guarana, or ginseng. In yet another embodiment of the invention, the supplement may also include vitamin D. The vitamin D may be in the form of D3 cholecalciferol.

In another aspect of the invention, a therapeutic effervescent composition may be used for treating a symptom or disorder associated with calcium imbalance. The therapeutic effervescent composition includes an effective amount of apoaequorin, an effective amount of vitamin D, at least one stimulant, and an effervescent system including at least one acid and at least one alkali compound. The at least one acidic component may be selected from citric acid, tartaric acid, malic acid, fumaric acid, adipic acid, succinic acids, or their salts. The at least one alkaline component may be selected from sodium carbonate, sodium bicarbonate, sodium sesquicarbonate, potassium carbonate, potassium bicarbonate, potassium sesquicarbonate, magnesium carbonate, sodium glycine carbonate, L-lysine carbonate, arginine carbonate, amorphous calcium carbonate, ammonium carbonate, or ammonium bicarbonate. The at least one stimulant may be selected from caffeine, yerba maté, ephedrine, guarana, or ginseng. The vitamin D may be in the form of D3 cholecalciferol.

In yet another aspect of the invention, a method for treating a symptom or disorder associated with calcium imbalance includes administering a therapeutic effervescent composition or supplement, containing apoaequorin and other components as discussed above, to a subject in need of such treatment, where apoaequorin is not administered with its cofactor, coelenterazine. The symptom or disorder associated with calcium imbalance may be sleep-related, energy-related, mood-related, pain-related, or memory-related. The administration of apoaequorin improves sleep quality, energy quality, mood quality, alleviates pain, or improves memory, as indicated by improved scores on a standardized cognitive assessment, in the subject. The symptom or disorder associated with calcium imbalance may further be related to neuronal excitability, muscle contraction, membrane permeability, cell division, hormone secretion, bone mineralization, or cell death following ischemia, in which the symptom or disorder improves upon administration of apoaequorin. A supplement or effervescent composition may be used to treat any of these symptoms or disorders associated with calcium imbalance. A supplement or effervescent composition may be used as a medicament.

Other objects, features and advantages of the present invention will become apparent after review of the specification and claims.

### DETAILED DESCRIPTION OF THE INVENTION

### I. IN GENERAL

Before the present materials and methods are described, it is understood that this invention is not limited to the particular methodology, and materials described, as these may vary. It is also to be understood that the terminology used herein is for the purpose of describing particular embodiments only, and is not intended to limit the scope of the present invention which will be limited only by the appended claims

It must be noted that as used herein and in the appended claims, the singular forms "a", "an", and "the" include plural reference unless the context clearly dictates otherwise. As well, the terms "a" (or "an"), "one or more" and "at least one" can be used interchangeably herein. It is also to be noted that the terms "comprising", "including", and "having" can be used interchangeably.

Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods and materials are now described.

### II. THE INVENTION

Aequorin is a photo-protein originally isolated from luminescent jellyfish and other marine organisms. The aequorin complex comprises a 22,285-dalton apoaequorin protein, molecular oxygen and the luminophore coelenterazine. When three Ca^{lf-} ions bind to this complex, coelenterazine is oxidized to coelentermide, with a concomitant release of carbon dioxide and blue light. Aequorin is not exported or secreted by cells, nor is it compartmentalized or sequestered within cells. Accordingly, aequorin measurements have been used to detect Ca²⁺ changes that occur over relatively long periods. In several experimental systems, aequorin's luminescence was detectable many hours to days after cell loading. It is further known that aequorin also does not disrupt cell functions or embryo development.

Because of its Ca^{2÷}-dependent luminescence, the aequorin complex has been extensively used as an intracellular Ca²⁺ indicator. *Aequorea victoria* aequorin has been specifically used to: (1) analyze the secretion response of single adrenal chromaffin cells to nicotinic cholinergic agonists; (2) clarify the role of Ca²⁺ release in heart muscle damage; (3) demonstrate the massive release of Ca²⁺ during fertilization; (4) study the regulation of the sarcoplasmic reticulum Ca²⁺ pump expression in developing chick myoblasts; and (5) calibrate micropipets with injection volumes of as little as three picoliters.

Apoaequorin has an approximate molecular weight of 22 kDa. Apoaequorin can be used to regenerate aequorin by reducing the disulfide bond in apoaequorin. The calcium-loaded apoaequorin retains the same compact scaffold and overall folding pattern as unreacted photoproteins containing a bound substrate.

Conventional purification of aequorin from the jellyfish *Aequorea victoria* requires laborious extraction procedures and sometimes yields preparations that are substantially heterogeneous or that are toxic to the organisms under study. Two tons of jellyfish typically yield approximately 125mg of the purified photoprotein. In contrast, recombinant aequorin is preferably produced by purifying apoaequorin from genetically engineered *Escherichia coli,* followed by reconstitution of the aequorin complex *in vitro* with pure coelenterazine. Apoaequorin useful in the present invention has been described and is commercially-obtainable through purification schemes and/or syntheses known to those of skill in the art. S. Inouye, S. Zenno, Y. Sakaki, and F. Tsuji. High level expression and purification of apoaequorin. (1991) Protein Expression and Purification 2, 122-126.

The present invention is directed to the administration of apoaequorin-containing effervescent compositions to a subject in order to correct or maintain the calcium balance in that subject. The maintenance of ionic calcium concentrations in plasma and body fluids is understood to be critical to a wide variety of bodily functions, including, but not limited to neuronal excitability, muscle contraction, membrane permeability, cell division, hormone secretion, bone mineralization, or the prevention of cell death following ischemia. Disruption in calcium homeostasis, i.e., a calcium imbalance, is understood to cause and/or correlate with many diseases, syndromes and conditions. Such diseases, syndromes and conditions include those associated with sleep quality, energy quality, mood quality, memory quality and pain perception. The study of CaBPs has led to their recognition as protective factors acting in the maintenance of proper ionic calcium levels.

The present invention is further directed to the administration of apoaequorin-containing effervescent compositions. A preferred embodiment of the apoaequorin-containing effervescent composition further contains at least one stimulant. Non-limiting examples of the at least one stimulant can include caffeine, yerba maté, ephedrine, guarana, and ginseng. In yet another preferred embodiment, the apoaequorin-containing effervescent composition may further contain vitamin D. In still yet another embodiment, a preferred embodiment of the apoaequorin-containing effervescent composition contains both at least one stimulant and vitamin D.

Vitamin D is a group of fat-soluble secosteroids responsible for increasing intestinal absorption of calcium, iron, magnesium, phosphate, and zinc. Vitamin D is produced by the body in response to the skin being exposed to ultraviolet rays from sunlight. It is also found in naturally occuring foods such as fish, fish liver oils, egg yolks, and in fortified dairy and grain products. In dietary supplements, the two most common compound forms of vitamin D are vitamin D3 (cholecalciferol) and vitamin D2 (ergocalciferol),

Vitamin D is a fat soluble vitamin that is biologically inert and must undergo two hydroxylations in the body for activation. The first occurs in the liver and converts vitamin D to 25-hydroxyvitamin D [25(OH)D], also known as calcifediol. The second occurs primarily in the kidney and forms the physiologically active 1,25-dihydroxyvitamin D [1,25(OH)2D], also known as calcitriol. The active form of vitamin D, calcitriol, circulates as a hormone in the blood, regulating the concentration of calcium and phosphate in the bloodstream and promoting the healthy growth and remodeling of bone.

Vitamin D promotes calcium absorption and maintains adequate serum calcium and phosphate concentrations to enable normal mineralization of bone and to prevent hypocalcemic tetany. It is also used for bone growth and bone remodeling by osteoblasts and osteoclasts. It has also been shown to play a role in modulation of cell growth, neuromuscular and immune function, and reduction of inflammation.

One preferred formulation of the present invention is directed to the administration of apoaequorin and vitamin D-containing effervescent compositions to a subject in order to correct or maintain the calcium balance and vitamin D levels in that subject. Vitamin D deficiency may contribute to calcium imbalances. The maintenance of ionic calcium concentrations in plasma and body fluids is understood to be critical to a wide variety of bodily functions, including, but not limited to neuronal excitability, muscle contraction, membrane permeability, cell division, hormone secretion, bone mineralization, or the prevention of cell death following ischemia. Disruption in calcium homeostasis, i.e., a calcium imbalance" is understood to cause and/or correlate with many diseases, syndromes and conditions. Such diseases, syndromes and conditions include those associated with sleep quality, energy quality, mood quality, and memory quality and pain perception. The study of CaBPs has led to their recognition as protective factors acting in the maintenance of proper ionic calcium levels.

The maintenance of vitamin D levels is understood to be critical to calcium absorption, modulation of cell growth, neuromuscular and immune function, and reduction of inflammation. Vitamin D deficiency is most associated with rickets, a disease in which the bone tissues does not properly mineralize, leading to soft bones and skeletal deformities. Guidelines from the Institute of Medicine provides that the recommended dietary allowance (RDA) of vitamin D is 600 international units (IU) for adults ages 1-70, and 800 IU for adults older than age 70 to optimize bone health.

Thus, incertain embodimerds, the methods of the present invention comprise administering apoaequorin (and optionally, vitamin D and/or at least one stimulant), in an effervescent formulation, as the active ingredient for treating calcium imbalance, for delaying the progression of calcium imbalance, for preventing the onset of calcium imbalance, and for preventing and/or treating the recurrence of calcium imbalance. In other embodiments, the invention provides methods which comprise administering apoacquorin in an effervescent formulation in combination with one or more additional agents having known therapeutic or nutraceutical value. Particularly preferred applications of apoaequorin are in treating one or more symptoms and disorders related to quality of sleep, energy, mood, memory and pain perception.

As used herein, the term "treating" includes preventative as well as disorder remittent treatment. As used herein, the terms "reducing", "alleviating", "suppressing" and "inhibiting" have their commonly understood meaning of lessening or decreasing. As used herein, the term "progression" means increasing in scope or severity, advancing, growing or becoming worse. As used herein, the term "recurrence" means the return of a disease after a remission.

As used herein, the term "administering" refers to bringing a patient, tissue, organ or cell in contact with apoaequorin. As used herein, administration can be accomplished *in vitro,* i.e., in a test tube, or *in vivo,* i.e., in cells or tissues of living organisms, for example, humans. In preferred embodiments, the present invention encompasses administering the effervescent formulations or compositions useful in the present invention to a patient or subject. A "patient" or "subject", used equivalently herein, refers to a mammal, preferably a human, that either: (1) has a calcium imbalance-related disorder remediable or treatable by administration of apoaequorin; or (2) is susceptible to a calcium imbalance-related disorder that is preventable by administering apoaequorin.

As used herein, the terms "effective amount" and "therapeutically effective amount" refer to the quantity of active agents sufficient to yield a desired therapeutic response without undue adverse side effects such as toxicity, irritation, or allergic response. The specific "effective amount" will, obviously, vary with such factors as the particular condition being treated, the physical condition of the patient, the type of animal being treated, the duration of the treatment, the nature of concurrent therapy (if any), and the specific formulations employed and the structure of the compounds or its derivatives. In this case, an amount would be deemed therapeutically effective if it resulted in one or more of the following: (1) the prevention of a calcium imbalance-related disorder; and (2) the reversal or stabilization of a calcium imbalance-related disorder. The optimum effective amounts can be readily determined by one of ordinary skill in the art using routine experimentation.

In certain preferred effervescent compositions for oral administration to subjects, apoaequorin is formulated in a pharmaceutical formulation at a dosage of approximately 10 mg/dose, with recommended dosage for a subject approximately 10 mg/day (i.e., one capsule per day). In other preferred apoaequorin containing effervescent compositions for oral administration, the composition or formulation further contains at least one stimulant.

To improve likelihood of ingestion and/or absorption of active pharmaceutical ingredients, pharmaceutical formulations containing effervescent components may be used. The formulation or composition can be in a solid form such as tablets, powders, capsules, pellets, that are further mixed with an aqueous vehicle before being orally digested. The compositions administrable by the invention can be prepared by known dissolving, mixing, granulating, or tablet-forming processes. For oral administration, apoaequorin or its physiologically-tolerated derivates such as salts, esters, N-oxides, and the like are mixed with additives customary for this purpose, such as vehicles, stabilizers, or inert diluents, and converted by customary methods into suitable forms for administration, such as tablets, coated tablets, hard or soft gelatin capsules, aqueous, alcoholic or oily solutions. The formulation is placed in an aqueous vehicle, such as an aqueous food or beverage, containing a minimal amount of water such as at least about 0.1 ml of water and may be stirred or agitated. The vehicle may be selected by the caregiver or chosen by the patient, or it may simply be the saliva and/or other water-containing fluid in the patient's mouth upon direct ingestion. Effervescent formulations, when added to a vehicle, generate a gas which causes effervescence and releases the active pharmaceutical ingredient(s) as the formulation breaks down in the vehicle. The vehicle is then orally ingested by the patient to administer the active pharmaceutical ingredient.

The term "effervescence" generally means the escape of a gas from a liquid or mixture (Hawley's Chemical Dictionary, pp. 432, 2001). Thus, the term "effervescent formulation", as used herein, is intended to generally refer to a composition or mixture of components that evolve one or more gases, under proper conditions, such as upon contact with water.

The term "aqueous vehicle", as used herein, is intended to refer to a medium or a carrier, such as a foodstuff, containing at least a minimal amount of water. Thus, the aqueous vehicle may be an oligohydrous vehicle containing a small amount of water, or it may be a vehicle having an abundance of water contained therein.

The term "foodstuff", as used herein, is intended to refer to any safe, consumable liquid, semi-solid, or solid substance. Thus, a foodstuff would include any beverage and any food, which may be consumed by mammals of all classes and ages.

To form effervescent formulations containing apoaequorin, the formulation can be placed within an inert vehicle such as tablets, powders, capsules, pellets. Examples of suitable inert vehicles are conventional tablet bases such as lactose, sucrose, or cornstarch in combination with binders such as acacia, cornstarch, gelatin, with disintegrating agents such as cornstarch, potato starch, alginic acid, or with a lubricant such as stearic acid or magnesium stearate.

Examples of suitable oily vehicles or solvents are vegetable or animal oils such as sunflower oil or fish-liver oil. Compositions can be effected both as dry and as wet granules. Examples are sterile liquids such as water and oils, with or without the addition of a surfactant and other pharmaceutically acceptable adjuvants. Illustrative oils are those of petroleum, animal, vegetable, or synthetic origin, for example, peanut oil, soybean oil or mineral oil. In general, water, saline, aqueous dextrose and related sugar solutions, and glycols such as propylene glycols or polyethylene glycol are preferred liquid carriers.

The gas which gives the effervescence is almost always carbon dioxide which derives from the reaction between an acid and a base. An effervescent system or effervescent couple therefore typically includes at least one acid and at least one alkali compound. Thus, the effervescent tablet consists of at least three components: (1) the active ingredient; (2) the acid; and (3) the alkali compound.

Many acidic and basic components are known to react in the presence of water to generate gas. For example, acids, such as citric acid, tartaric acid, malic acid, fumaric acid, adipic acid, succinic acids, and the like, and combinations thereof are reactive with carbonates, or a source thereof, in water to generate CO2 gas. Suitable acids include, without limitation, food acids, acid anhydrides and acid salts. Examples of food acids include citric acid, tartaric acid, malic acid, fumaric acid, adipic acid, succinic acids, and the like. Anhydrides of the above-described acids may also be used as anhydrides generally degrade in the presence of water to generate the reactive acid. Acid salts generally disassociate in water, or in the water content of the aqueous vehicle, to provide the reactive acid species. Examples of suitable acid salts include, without limitation, sodium dihydrogen phosphate, disodium dihydrogen pyrophosphate, acid citrate salts and sodium acid sulfite. The overall solubility of the acid, or source thereof, in water will vary is appreciated by those skilled in the art. The effectiveness of the acid in generating the gas, and the amount of gas generated, is generally dependent on water solubility of the acid form in the effervescent formulation.

Suitable sources of carbonate include, without limitation, dry solid carbonate, bicarbonate, and sesqui-bicarbonate salts of metals, such as sodium, potassium, lithium, calcium, and magnesium. Examples of suitable carbonates include, without limitation, sodium carbonate, sodium bicarbonate, sodium sesquicarbonate, potassium carbonate, potassium bicarbonate, potassium sesquicarbonate, magnesium carbonate, sodium glycine carbonate, L-lysine carbonate, arginine carbonate, and amorphous calcium carbonate. Ammonium carbonate and ammonium bicarbonate are also suitable carbonates. In addition, any combination of the above sources of carbonate may be used as the basic component in the formulation. Excess basic component provides advantages, including providing a basic vehicle and/or a basic oral environment, taste masking properties, and many other benefits. In an example of pharmaceutical formulations containing effervescent components, the gas-generating effervescent component should generally comprise about 5% to about 85% of the total weight of the composition.

Compositions of effervescent tablets may also include a lubricant is preferably selected from water-soluble compounds to form a clear solution. Examples of this kind of lubricants are sodium benzoate, sodium acetate, fumaric acid, polyethylenglycols (PEG) higher than 4000, alanine, and glycine.

Conventional excipients such as diluents, ligands, bufferings, sweeteners, flavorings, colorings, solubilizers, disintegrants, wetting agents and other excipients of common use may be added to the formulation. Suitable flavoring agents include natural flavors, artificial flavors, and mints, such as peppermint, menthol, artificial vanilla, cinnamon, various fruit flavors, both individual and mixed, and the like are contemplated. The flavorings are generally utilized in amounts that will vary depending upon the individual flavor, and may, for example, range in amounts of about 0.5 to about 3% by weight of the final composition weight.

In the instance where sweeteners are utilized, the present invention contemplates the inclusion of those sweeteners well known in the art, including both natural and artificial sweeteners. Thus, additional sweeteners may be chosen from the following non-limiting list: sugars such as sucrose, glucose (corn syrup), invert sugar, fructose, and mixtures thereof; saccharin and its various salts such as the sodium or calcium salt; cyclamic acid and its various salts such as the sodium salt; the dipeptide sweeteners such as aspartame; dihydrochalcone; glycyrrhizin; Stevia rebaudiana (Stevioside); and sugar alcohols such as sorbitol, sorbitol syrup, mannitol, xylitol, and the like. Also contemplated as an additional sweetener is the nonfermentable sugar substitute (hydrogenated starch hydrolysate) which is described in U.S. Pat. No. Re 26,959. Also contemplated is the synthetic sweetener 3,6-dihydro-6-methyl-1-1-2,3-oxathiazin-4-one-2,2-dioxide particularly the potassium (Acesulfame-K), sodium and calcium salts thereof as described in German Pat. No. 2,001,017.7. In general, the amount of sweetener will vary according to the type of sweetener and the desired taste of the final product. For example, natural sweeteners may be used in amounts up to about 5% by weight, while artificial sweeteners may be in amounts up to about 1% by weight.

The colorants useful in the present invention include pigments, such as titanium dioxide, that may be incorporated in amounts of up to about 1% by weight, and preferably up to about 0.6% by weight. Also, the colorants may include other dyes suitable for food, drug and cosmetic applications, and known as F.D. & C. dyes and the like. The materials acceptable for the foregoing spectrum of use are preferably water-soluble. Illustrative examples include indigoid dye, known as F.D. & C. Blue No. 2, which is the disodium salt of 5,5'-indigotin disulfonic acid. Similarly, the dye known as F.D. & C. Green No. 1, comprises a triphenylmethane dye and is the monosodium salt of 4-[4-N ethyl-p-sulfobenzylamino)diphenylmethylene][1-(N-ethyl-N-p-sulfoniumbenzyl)-2,5-cyclohexadienimine]. A full recitation of all F.D. & C. and D. & C. dyes and their corresponding chemical structures may be found in the Kirk-Othmer Encyclopedia of Chemical Technology, in Volume 5, at Pages 857-884.

Pharmaceutical formulation compositions may further include liquids or lyophilized or otherwise dried formulations and include diluents of various buffer content (e.g., Tris-HC1, acetate, phosphate), pH and ionic strength, additives such as albumin or gelatin to prevent absorption to surfaces, detergents (e.g., Tween 20, Tween 80, Pluronic F68, bile acid salts), solubilizing agents (e.g., glycerol, polyethylene glycerol), anti-oxidants (e.g., ascorbic acid, sodium metabisulfite), preservatives (e.g., Thimerosal, benzyl alcohol, parabens), bulking substances or tonicity modifiers (e.g., lactose, mannitol), covalent attachment of polymers such as polyethylene glycol to the protein, complexation with metal ions, or incorporation of the material into or onto particulate preparations of polymeric compounds such as polylactic acid, polyglycolic acid, or hydrogels, or onto liposomes, microemulsions, micelles, lamellar or multilamellar vesicles, erythrocyte ghosts or spheroplasts. Such compositions will influence the physical state, solubility, stability, rate of *in vivo* release, and rate of *in vivo* clearance. Controlled or sustained release compositions include formulation in lipophilic depots (e.g., fatty acids, waxes, oils).

Chemical entities modified by the covalent attachment of water-soluble polymers such as polyethylene glycol, copolymers of polyethylene glycol and polypropylene glycol, carboxymethyl cellulose, dextran, polyvinyl alcohol, polyvinylpyrrolidone or polyproline are known to exhibit substantially longer half-lives in blood following intravenous injection than do the corresponding unmodified compounds. Such modifications may also increase the chemical entities solubility in aqueous solution, eliminate aggregation, enhance the physical and chemical stability of the compound, and greatly reduce the immunogenicity and reactivity of the compound. As a result, the desired *in vivo* biological activity may be achieved by the administration of such polymer-entity abducts less frequently or in lower doses than with the unmodified entity.

The preparation of compositions which contain an active component is well understood in the art. The active therapeutic ingredient is often mixed with excipients which are pharmaceutically acceptable and compatible with the active ingredient. Suitable excipients include, for example, water, saline, dextrose, glycerol, ethanol, or the like or any combination thereof. In addition, the composition can contain minor amounts of auxiliary substances such as wetting or emulsifying agents, pH buffering agents which enhance the effectiveness of the active ingredient.

An active component can be formulated into the effervescent composition as neutralized pharmaceutically acceptable salt forms. Pharmaceutically acceptable salts include the acid addition salts, which are formed with inorganic acids such as, for example, hydrochloric, or phosphoric acids, or such organic acids as acetic, tartaric, mandelic, and the like. Salts formed from the free carboxyl groups can also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

Salts of apoaequorin are preferably pharmaceutically acceptable salts. Other salts may, however, be useful in the preparation of the effervescent compositions according to the invention or of their pharmaceutically acceptable salts. Suitable pharmaceutically acceptable salts include acid addition salts which may, for example, be formed by mixing a solution of apoaequorin with a solution of a pharmaceutically acceptable acid such as hydrochloric acid, sulphuric acid, methanesulphonic acid, fumaric acid, maleic acid, succinic acid, acetic acid, benzoic acid, oxalic acid, citric acid, tartaric acid, carbonic acid or phosphoric acid.

In addition, apoaequorin-containing effervescent compositions described herein may be provided in the form of nutraceutical compositions where apoaequorin prevents the onset of or reduces or stabilizes various deleterious calcium imbalance-related disorders. The term "nutraceutical" or "nutraceutical composition", for the purpose of this specification, refers to a food item, or a part of a food item, that offers medical health benefits, including prevention and/or treatment of disease. A nutraceutical composition according to the present invention may contain only apoaequorin as an active ingredient, or alternatively, may further comprise, in admixture with dietary supplements including vitamins, co-enzymes, minerals, herbs, amino acids and the like which supplement the diet by increasing the total intake of that substance.

Therefore, the present invention provides methods of providing nutraceutical benefits to a patient comprising the step of administering to the patient a nutraceutical effervescent composition containing apoaequorin. Such compositions generally include a "nutraceutically-acceptable carrier" which, as referred to herein, is any carrier suitable for oral delivery including aforementioned pharmaceutically-acceptable carriers suitable for the oral route. Thus, the invention provides a more favorable mechanism for orally delivering a medicament to a patient, which the patient may not have otherwise ingested, in a medium more convenient and patient-friendly than previously delivered.

In certain embodiments, nutraceutical effervescent compositions according to the invention comprise dietary supplements which, defined on a functional basis, include immune boosting agents, anti-inflammatory agents, anti-oxidant agents, anti-viral agents, or mixtures thereof.

Immune boosters and/or anti-viral agents are useful for accelerating wound- healing and improved immune function; and they include extracts from the coneflowers, or herbs of the genus *Echinacea,* extracts from herbs of the genus *Sambuca,* and Goldenseal extracts. Herbs of the genus *Astragalus* are also effective immune boosters in either their natural or processed forms. *Astragalus* stimulates development of stem cells in the marrow and lymph tissue active immune cells. Zinc and its bioactive salts, such as zinc gluconate and zinc acetate, also act as immune boosters in the treatment of the common cold.

Antioxidants include the natural, sulfur-containing amino acid allicin, which acts to increase the level of antioxidant enzymes in the blood. Herbs or herbal extracts, such as garlic, which contain allicin, are also effective antioxidants. The catechins, and the extracts of herbs such as green tea containing catechins, are also effective antioxidants. Extracts of the genus *Astragalus* also show antioxidant activity. The bioflavonoids, such as quercetin, hesperidin, rutin, and mixtures thereof, are also effective as antioxidants. The primary beneficial role of the bioflavonoids may be in protecting vitamin C from oxidation in the body. This makes more vitamin C, or ascorbic acid, available for use by the body.

Bioflavonoids such as quercetin are also effective anti-inflammatory agents, and may be used as such in the inventive compositions. Anti-inflammatory herbal supplements and anti-inflammatory compounds derived from plants or herbs may also be used as anti-inflammatory agents in the inventive composition. These include bromolain, a proteolytic enzyme found in pineapple; teas and extracts of stinging nettle; turmeric, extracts of turmeric, or curcumin, a yellow pigment isolated from turmeric.

Another supplement which may be used in the present invention is ginger, derived from herbs of the genus *Zingiber.* This has been found to possess cardiotonic activity due to compounds such as gingerol and the related compound shogaol as well as providing benefits in the treatment of dizziness, and vestibular disorders. Ginger is also effective in the treatment of nausea and other stomach disorders.

Supplements which assist in rebuilding soft tissue structures, particularly in rebuilding cartilage, are useful in compositions for treating the pain of arthritis and other joint disorders. Glucosamine, glucosamine sulfate, chondroitin may be derived from a variety of sources such as Elk Velvet Antler. Marine lipid complexes, omega 3 fatty acid complexes, and fish oil are also known to be useful in treating pain associated with arthritis.

Supplements useful in treating migraine headaches include feverfew and *Gingko biloba.* The main active ingredient in feverfew is the sesquiterpene lactone parthenolide, which inhibits the secretions of prostaglandin which in turn cause pain through vasospastic activity in the blood vessels. Feverfew also exhibits anti-inflammatory properties. Fish oil, owing to its platelet-stabilizing and antivasospastic actions, may also be useful in treating migraine headaches. The herb *Gingko biloba* also assists in treatment of migraines by stabilizing arteries and improving blood circulation.

Although some of the supplements listed above have been described as to their pharmacological effects, other supplements may also be utilized in the present invention and their effects are well documented in the scientific literature.

The invention will be more fully understood upon consideration of the following non-limiting Examples.

### III. EXAMPLES

### Example 1. Preferred Formulation of an Apoaequorin-Containing Effervescent Composition

This example describes a particularly preferred apoaequorin-containing composition including 13.8 mg of apoaequorin, 27.8 mg of vitamin D3 (cholecalciferol) dry 100,000 IU/g, 135 mg of caffeine anhydrous granular EP, 500 mg of sorbitol BP, 417 of xylitol DC, 80 mg of maize starch, 2 mg of riboflavin 5-phosphate sodium, 45 mg of sodium saccharin BP , 250 mg of (nat) orange flavor, 40 mg of redbeet powder, 1450 mg of citric acid anhydros, and 1040 mg of sodium bicarbonate. The xylitol DC component contains xylitol and sodium carboxymethylcellulose. The Vitamin D3 component contains sucrose, acacia, corn starch, medium chain triglycerides, silicon dioxide, vitamin D3, and DL alpha tocopherol. The (nat) orange flavor component contains maltodextrin, flavoring preparations, silicon dioxide, natural flavoring substances, and vitamin E. The red beet powder component contains beetroot juice concentrate, maltodextrin, and citric acid. The total weight of the tablet is 4,000 mg.

| Name of Material | Mg per Tablet |
|---|---|
| Apoaequorin | 13.8 |
| Vitamin D3 (cholecalciferol) Dry 100,000 IU/g | 27.8 |
| *Vitamin D3 contains sucrose, acacia, corn starch, medium chain triglycerides, silicon dioxide, vitamin D3, and DL alpha tocopherol. | |
| Caffeine Anhydrous Granular EP | 135 |
| Sorbitol BP | 500 |
| Xylitol DC | 417 |
| * Xylitol DC contains xylitol and sodium carboxymethylcellulose | |
| Maize Starch | 80 |
| Riboflavin 5-Phosphate Sodium | 2 |
| Sodium Saccharin BP | 45 |
| (Nat) Orange Flavor | 250 |
| * (Nat) orange flavor contains maltodextrin, flavoring preparations, silicon dioxide, natural flavoring substances, and vitamin E. | |
| Red Beet Powder | 40 |
| * Red beet powder contains beetroot juice concentrate, maltodextrin, and citric acid | |
| Citric Acid Anhydrous | 1450 |
| Sodium Bicarbonate | 1040 |
| Tablet Weight: 4,000 mg | |

## Claims

1. A supplement in form of an effervescent tablet for treating a symptom or disorder associated with calcium imbalance comprising an effective amount of apoaequorin and an effervescent couple, the couple comprising an acidic component and an alkaline component;
wherein the effervescent component comprises about 5% to about 85% of the total weight of the effervescent tablet.

2. The supplement of claim 1 wherein the supplement further includes at least one stimulant.

3. A therapeutic effervescent composition for treating a symptom or disorder associated with calcium imbalance comprising:
an effective amount of apoaequorin;
an effective amount of vitamin D;
at least one stimulant; and
an effervescent system including at least one acid and at least one alkali compound;
wherein the effervescent component comprises about 5% to about 85% of the total weight of the composition.

4. The supplement or therapeutic effervescent composition of claim 2 or 3 wherein the at least one stimulant is selected from caffeine, yerba maté, ephedrine, guarana, or ginseng.

5. The supplement or therapeutic effervescent composition of any one of claims 1-4 wherein the acidic component is selected from citric acid, tartaric acid, malic acid, fumaric acid, adipic acid, succinic acids, or their salts.

6. The supplement or therapeutic effervescent composition of any one of claims 1-5 wherein the alkaline component is selected from sodium carbonate, sodium bicarbonate, sodium sesquicarbonate, potassium carbonate, potassium bicarbonate, potassium sesquicarbonate, magnesium carbonate, sodium glycine carbonate, L-lysine carbonate, arginine carbonate, amorphous calcium carbonate, ammonium carbonate, or ammonium bicarbonate.

7. A supplement according to any one of claims 1, 2 or 4-6, wherein the supplement further includes vitamin D.

8. A therapeutic effervescent composition according to any one of claims 3-6, or a supplement according to claim 7, wherein the vitamin D is in the form of D3 cholecalciferol.

9. A supplement or therapeutic effervescent composition according to any one of claims 1 to 8, for use in medicine.

10. A supplement or therapeutic effervescent composition according to any one of claims 1 to 8, for use in treating a symptom or disorder associated with calcium imbalance in a subject in need of such treatment, wherein in said use said apoaequorin is not administered with its cofactor, coelenterazine.

11. A supplement or therapeutic effervescent composition for use according to claim 10, wherein:
i) the symptom or disorder associated with calcium imbalance is sleep-related and the administration of apoaequorin to said subject improves sleep quality in the subject; or
ii) the symptom or disorder associated with calcium imbalance is energy-related and administration of apoaequorin to said subject improves energy quality in the subject; or
iii) the symptom or disorder associated with calcium imbalance is mood-related and administration of apoaequorin to said subject improves mood quality in the subject; or
iv) the symptom or disorder associated with calcium imbalance is pain-related and administration of apoaequorin to said subject alleviates pain in the subject; or
v) the symptom or disorder associated with calcium imbalance is memory-related and administration of apoaequorin to said subject improves memory, as indicated by improved scores on a standardized cognitive assessment; or
vi) the symptom or disorder associated with calcium imbalance is related to neuronal excitability, muscle contraction, membrane permeability, cell division, hormone secretion, bone mineralization, or cell death following ischemia.

12. A supplement or effervescent composition according to any one of claims 1-8 for use in improving sleep quality, energy quality, mood quality, or memory in a subject in need thereof.

13. A supplement or effervescent composition according to any one of claims 1-8 for use in alleviating pain in a subject in need thereof.

14. A supplement or effervescent composition according to any one of claims 1-8 for use in treating a symptom or disorder associated with calcium imbalance related to neuronal excitability, muscle contraction, membrane permeability, cell division, hormone secretion, bone mineralization, or cell death following ischemia.

## Patentansprüche

1. Ergänzungsmittel in Form einer Brausetablette zum Behandeln eines Symptoms oder einer Erkrankung, das/die mit einem Kalziumungleichgewicht assoziiert ist, umfassend eine wirksame Menge an Apoaequorin und ein Brausepaar, wobei das Paar eine saure Komponente und eine alkalische Komponente umfasst;
wobei die Brausekomponente etwa 5 % bis etwa 85 % des Gesamtgewichts der Brausetablette ausmacht.

2. Ergänzungsmittel nach Anspruch 1, wobei das Ergänzungsmittel ferner wenigstens ein Stimulans beinhaltet.

3. Therapeutische Brausezusammensetzung zum Behandeln eines Symptoms oder einer Erkrankung, die mit einem Kalziumungleichgewicht assoziiert ist, umfassend:
eine wirksame Menge an Apoaequorin;
eine wirksame Menge an Vitamin D;
wenigstens ein Stimulans; und
ein Brausesystem, das wenigstens eine Säure und wenigstens eine alkalische Verbindung beinhaltet;
wobei die Brausekomponente etwa 5 % bis etwa 85 % des Gesamtgewichts der Zusammensetzung ausmacht.

4. Ergänzungsmittel oder therapeutische Brausezusammensetzung nach Anspruch 2 oder 3, wobei das wenigstens eine Stimulans aus Koffein, Mate, Ephedrin, Guarana oder Ginseng ausgewählt ist.

5. Ergänzungsmittel oder therapeutische Brausezusammensetzung nach einem der Ansprüche 1-4, wobei die Säurekomponente aus Zitronensäure, Weinsäure, Apfelsäure, Fumarsäure, Adipinsäure, Bernsteinsäure oder deren Salzen ausgewählt ist.

6. Ergänzungsmittel oder therapeutische Brausezusammensetzung nach einem der Ansprüche 1-5, wobei die Alkalikomponente aus Natriumcarbonat, Natriumbicarbonat, Natriumsesquicarbonat, Kaliumcarbonat, Kaliumbicarbonat, Kaliumsesquicarbonat, Magnesiumcarbonat, Natriumglycincarbonat, L-Lysincarbonat, Arginincarbonat, amorphem Calciumcarbonat, Ammoniumcarbonat oder Ammoniumbicarbonat ausgewählt ist.

7. Ergänzungsmittel nach einem der Ansprüche 1, 2 oder 4-6, wobei das Ergänzungsmittel ferner Vitamin D beinhaltet.

8. Therapeutische Brausezusammensetzung nach einem der Ansprüche 3-6 oder Ergänzungsmittel nach Anspruch 7, wobei das Vitamin D in Form von D3-Cholecalciferol vorliegt.

9. Ergänzungsmittel oder therapeutische Brausezusammensetzung nach einem der Ansprüche 1 bis 8, für die Verwendung in der Medizin.

10. Ergänzungsmittel oder therapeutische Brausezusammensetzung nach einem der Ansprüche 1 bis 8, für die Verwendung bei dem Behandeln eines Symptoms oder einer Erkrankung, das/die mit einem Kalziumungleichgewicht assoziiert ist, bei einem Subjekt, das einer solchen Behandlung bedarf, wobei bei der Verwendung das Apoaequorin nicht zusammen mit seinem Cofaktor, Coelenterazin, verabreicht wird.

11. Ergänzungsmittel oder therapeutische Brausezusammensetzung für die Verwendung nach Anspruch 10, wobei:
i) das Symptom oder die Erkrankung, das/die mit einem Kalziumungleichgewicht assoziiert ist, schlafbezogen ist und die Verabreichung von Apoaequorin an das Subjekt die Schlafqualität des Subjekts verbessert; oder
ii) das Symptom oder die Erkrankung, das/die mit einem Kalziumungleichgewicht assoziiert ist, energiebezogen ist und die Verabreichung von Apoaequorin an das Subjekt die Energiequalität des Subjekts verbessert; oder
iii) das Symptom oder die Erkrankung, das/die mit einem Kalziumungleichgewicht assoziiert ist, stimmungsbezogen ist und die Verabreichung von Apoaequorin an das Subjekt die Stimmungsqualität des Subjekts verbessert; oder
iv) das Symptom oder die Erkrankung, das/die mit einem Kalziumungleichgewicht assoziiert ist, schmerzbezogen ist und die Verabreichung von Apoaequorin an das Subjekt die Schmerzen des Subjekts lindert; oder
v) das Symptom oder die Erkrankung, das/die mit einem Kalziumungleichgewicht assoziiert ist, gedächtnisbezogen ist und die Verabreichung von Apoaequorin an das Subjekt das Gedächtnis verbessert, wie durch verbesserte Bewertungsziffern bei einer standardisierten kognitiven Beurteilung angegeben; oder
vi) das Symptom oder die Erkrankung, das/die mit einem Kalziumungleichgewicht assoziiert ist, mit neuronaler Erregbarkeit, Muskelkontraktion, Membranpermeabilität, Zellteilung, Hormonausschüttung, Knochenmineralisierung oder Zelltod infolge einer Ischämie in Zusammenhang steht.

12. Ergänzungsmittel oder Brausezusammensetzung nach einem der Ansprüche 1-8 für die Verwendung bei der Verbesserung der Schlafqualität, der Energiequalität, der Stimmungsqualität oder des Gedächtnisses des Subjekts, das dessen bedarf.

13. Ergänzungsmittel oder Brausezusammensetzung nach einem der Ansprüche 1-8 für die Verwendung bei dem Lindern von Schmerzen eines Subjekts, das dessen bedarfs.

14. Ergänzungsmittel oder Brausezusammensetzung nach einem der Ansprüche 1-8 für die Verwendung bei dem Behandeln eines Symptoms oder einer Erkrankung, das/die mit einem Kalziumungleichgewicht assoziiert ist und mit neuronaler Erregbarkeit, Muskelkontraktion, Membranpermeabilität, Zellteilung, Hormonausschüttung, Knochenmineralisierung oder Zelltod infolge einer Ischämie in Zusammenhang steht.

## Revendications

1. Complément sous forme d'un comprimé effervescent destiné au traitement d'un symptôme ou d'un trouble associé à un déséquilibre calcique, comprenant une quantité efficace d'apoaéquorine et un couple effervescent, le couple comprenant un composant acide et un composant alcalin ;
le composant effervescent représentant d'environ 5 % à environ 85 % du poids total du comprimé effervescent.

2. Complément selon la revendication 1, dans lequel le complément comprend en outre au moins un stimulant.

3. Composition effervescente thérapeutique destinée au traitement d'un symptôme ou d'un trouble associé à un déséquilibre calcique, comprenant :
une quantité efficace d'apoaéquorine,
une quantité efficace de vitamine D,
au moins un stimulant, et
un système effervescent comprenant au moins un acide et au moins un composé alcalin ;
le composant effervescent représentant d'environ 5 % à environ 85 % du poids total de la composition.

4. Complément ou composition effervescente thérapeutique selon la revendication 2 ou 3, dans lesquels ledit au moins un stimulant est choisi parmi la caféine, le maté, l'éphédrine, le guarana ou le ginseng.

5. Complément ou composition effervescente thérapeutique selon l'une quelconque des revendications 1 à 4, dans lesquels le composant acide est choisi parmi l'acide citrique, l'acide tartrique, l'acide malique, l'acide fumarique, l'acide adipique, les acides succiniques, ou leurs sels.

6. Complément ou composition effervescente thérapeutique selon l'une quelconque des revendications 1 à 5, dans lesquels le composé alcalin est choisi parmi le carbonate de sodium, le bicarbonate de sodium, le sesquicarbonate de sodium, le carbonate de potassium, le bicarbonate de potassium, le sesquicarbonate de potassium, le carbonate de magnésium, le carbonate de glycine sodique, le carbonate de L-lysine, le carbonate d'arginine, le carbonate de calcium amorphe, le carbonate d'ammonium et/ou le bicarbonate d'ammonium.

7. Complément selon l'une quelconque des revendications 1, 2 ou 4 à 6, ledit complément comprenant en outre de la vitamine D.

8. Composition effervescente thérapeutique selon l'une quelconque des revendications 3 à 6, ou complément selon la revendication 7, dans lesquels la vitamine D est sous la forme de cholécalciférol D3.

9. Complément ou composition effervescente thérapeutique selon l'une quelconque des revendications 1 à 8, destinés à être utilisés en médecine.

10. Complément ou composition effervescente thérapeutique selon l'une quelconque des revendications 1 à 8, destinés à être utilisés dans le traitement d'un symptôme ou d'un trouble associé à un déséquilibre calcique chez un sujet ayant besoin d'un tel traitement, étant entendu que, dans le cadre de ladite utilisation, l'apoaéquorine n'est pas administrée avec son cofacteur, la coelentérazine.

11. Complément ou composition effervescente thérapeutique destinés à être utilisés selon la revendication 10, dans lesquels :
i) le symptôme ou le trouble associé à un déséquilibre calcique est lié au sommeil et l'administration d'apoaéquorine audit sujet améliore la qualité du sommeil chez le sujet, ou
ii) le symptôme ou le trouble associé à un déséquilibre calcique est lié à l'énergie et l'administration d'apoaéquorine audit sujet améliore la qualité de l'énergie chez le sujet ; ou
iii) le symptôme ou le trouble associé à un déséquilibre calcique est lié à l'humeur et l'administration d'apoaéquorine audit sujet améliore la qualité de l'humeur chez le sujet ; ou
iv) le symptôme ou le trouble associé à un déséquilibre calcique est lié à la douleur et l'administration d'apoaéquorine audit sujet atténue la douleur chez le sujet ; ou
v) le symptôme ou le trouble associé à un déséquilibre calcique est lié à la mémoire et l'administration d'apoaéquorine audit sujet améliore la mémoire, comme l'indiquent des scores améliorés dans le cadre d'une évaluation cognitive normalisée ; ou
vi) le symptôme ou le trouble associé à un déséquilibre calcique est lié à l'excitabilité neuronale, à la contraction musculaire, à la perméabilité membranaire, à la division cellulaire, à la sécrétion hormonale, à la minéralisation osseuse ou à la mort cellulaire à la suite d'une ischémie.

12. Complément ou composition effervescente selon l'une quelconque des revendications 1 à 8, destinés à être utilisés dans l'amélioration de la qualité du sommeil, la qualité de l'énergie, la qualité de l'humeur et/ou la mémoire chez un sujet en ayant besoin.

13. Complément ou composition effervescente selon l'une quelconque des revendications 1 à 8, destinés à être utilisés pour atténuer la douleur chez un sujet en ayant besoin.

14. Complément ou composition effervescente selon l'une quelconque des revendications 1 à 8, destinés à être utilisés dans le traitement d'un symptôme ou d'un trouble associé à un déséquilibre calcique lié à l'excitabilité neuronale, à la contraction musculaire, à la perméabilité membranaire, à la division cellulaire, à la sécrétion hormonale, à la minéralisation osseuse et/ou à la mort cellulaire à la suite d'une ischémie.
